# EUROPEAN PATENT SPECIFICATION

(11) **EP 3 362 405 B1**
(45) Date of publication and mention of the grant of the patent: **02.11.2022**
(21) Application number: 16801322.5
(22) Date of filing: 17.10.2016
(51) Int. Cl.: C01B 3/38, C07C 29/151, C07C 31/04

(54) **PROCESS AND PRODUCTION UNIT FOR THE PRODUCTION OF METHANOL**
VERFAHREN UND PRODUKTIONSEINHEIT ZUR HERSTELLUNG VON METHANOL
PROCÉDÉ DE PRODUCTION ET UNITÉ DE PRODUCTION DE MÉTHANOL

(30) Priority: 15.10.2015 EP 15190011
(43) Date of publication of application: 22.08.2018
(73) Proprietor: Stamicarbon B.V. acting under the name of MT Innovation Center, 6135 KW Sittard (NL)
(72) Inventor: IAQUANIELLO, Gaetano, I-00148 Rome (IT); SALLADINI, Annarita, I-00148 Rome (IT)
(74) Representative: V.O.
(86) International application number: PCT/NL2016/050711
(87) International publication number: WO 2017/065613

(56) References cited:
- WO-A1-99/15483
- WO-A1-2013/062413
- NARENDRA DAVE ET AL: "Comparative Assessment of Catalytic Partial Oxidation and Steam Reforming for the Production of Methanol from Natural Gas", INDUSTRIAL & ENGINEERING CHEMISTRY RESEARCH., vol. 34, no. 4, 1 April 1995 (1995-04-01), pages 1037-1043, XP055247983, US ISSN: 0888-5885, DOI: 10.1021/ie00043a005
- "Technology for a better society SINTEF FACTS The Remote Gas Project Completed", , 1 April 2010 (2010-04-01), pages 1-4, XP055248002, Retrieved from the Internet: URL:https://www.sintef.no/globalassets/pro ject/oilandgas/pdf/remote_gas.pdf [retrieved on 2016-02-05]
- Gaetano Iaquaniello ET AL: "Natural Gas Catalytic Partial Oxidation: A Way to Syngas and Bulk Chemicals Production" In: "Natural Gas - Extraction to End Use", 31 October 2012 (2012-10-31), InTech, XP055247808, ISBN: 978-953-51-0820-7 DOI: 10.5772/48708, Chapter 3, in particular 3.4; the whole document
- Ji Yaying ET AL: "Partial oxidation of methane with air or 02 and steam to syngas on a Ni-based catalyst", Journal of Natural Gas Chemistry, 1 January 2000 (2000-01-01), pages 291-341, XP055248175, Retrieved from the Internet: URL:http://www.jenergychem.org/fileup/PDF/ 2000-04-0291.pdf [retrieved on 2016-02-05]

## Description

### Field of the invention

The present invention relates to the production of methanol from a hydrocarbon feed, as set out in the claims. In particular, the present invention relates to the off-shore production of methanol. Also, the invention pertains to the production of a synthesis gas suitable for the production of methanol.

### Background of the invention

Commercial methanol plants produce methanol in several steps, usually including synthesis gas preparation (reforming), methanol synthesis and methanol purification. Since these steps are conducted in separate process sections, the technology for each section can be selected and optimized independently. The usual criteria for the selection of technology are capital cost and plant efficiency. The preparation of synthesis gas and compression typically accounts for about 60% of the total investment, and almost all energy is consumed in this process section. Therefore, the technology to produce synthesis gas is of major importance.

The synthesis gas for the production of methanol is usually obtained by subjecting a desulfurized hydrocarbon feed to steam reforming (SR) at a temperature from 800 to 950°C in the presence of a fixed bed of catalyst, typically containing nickel. The resulting synthesis gas is cooled and compressed to be used further in the methanol process. However, the synthesis gas obtained in steam reforming is usually characterized by a too low carbon/hydrogen ratio compared to a stoichiometric composition optimal for methanol synthesis. As a result, the methanol synthesis reactor typically operates at a large hydrogen excess which results in an overall low plant efficiency.

In particular, methanol synthesis gas can conventionally be characterized by a molar ratio (H₂ - CO₂) / (CO + CO₂). This ratio is referred to herein as an R ratio. An R ratio equal to 2 corresponds to a stoichiometric synthesis gas for formation of methanol.

Most current plants for producing methanol use natural gas as feed with a limited amount of CO₂. CO₂ is normally removed in a dedicated gas treatment plant together with other components such as H₂S before its use.

Synthesis gas obtained in steam reforming is usually characterized by a too low carbon/hydrogen ratio compared to a stoichiometric optimal composition. In the Combined Reforming Technology (CRT), described in EP023307, the hydrocarbon feed is split into two fractions. One of these fractions is subjected to primary reforming and is then combined with the second fraction, after which both are routed to a secondary reforming.

The known processes are not suitable for off-shore (floating) methanol production. This refers to production on vessels, which is known as Floating Production Storage and Offloading (FPSO). Steam reformers are large and heavy items, and also other systems are involved (refractory systems and unrestrained tube systems) that are not suitable for the movements to which an FPSO can be subjected. The typical very large fired heater necessary for auto thermal reformer is also not suited for such movement.

In another patent application, WO 2013/062413 A1, the technology of Catalytic Partial Oxidation (CPO) is used to produce a synthesis gas having an optimal components ratio for methanol production. However, hereby the synthesis gas coming from CPO needs a further treatment, viz. a water-gas shift reaction (WGS) and pressure swing absorption (PSA) before reaching the target ratio. This further treatment is not suitable for floating production of methanol. Also in the process of WO 2013/062413, it is thereby taught that, in order to be suitable for methanol production, the synthesis gas as obtained preferably has an R ratio in a range close to 2. To this end, WO 2013/062413 uses synthesis gas obtained from hydrocarbons substantially without carbon dioxide.

Narendra et al (INDUSTRIAL & ENGINEERING CHEMISTRY RESEARCH., vol. 34, no. 4, 1995, pages 1037-1043) discloses combining a CPO reactor with a methanol synthesis reactor and the effect of different reaction parameters, such as steam feed to the CPO reactor, on the CPO reaction.

It is desired to provide a method for producing synthesis gas for methanol production, which process would be substantially devoid of the aforementioned disadvantages. Particularly, it is desired to have a process which would be capable of handling a natural gas having a high level of CO₂ without the need of CO₂ removal. Preferably, a process is sought that would be suitable for FPSO.

### Summary of the invention

In order to better address one or more of the foregoing desires, the invention presents, in one aspect, a process for the production of methanol from a hydrocarbon feed, the process comprising the steps of
(a) providing a hydrocarbon feed;
(b) subjecting the hydrocarbon feed to Catalytic Partial Oxidation (CPO) so as to obtain a synthesis gas mixture;
(c) subjecting the synthesis gas mixture to methanol-forming conditions together with an additonal feed of hydrogen (H₂), so as to obtain methanol;
wherein the hydrocarbon feed is a CO₂-containing hydrocarbon feed and wherein the Catalytic Partial Oxidation is conducted in the presence of a molar excess of H₂O, and wherein wherein the additional hydrogen feed is obtained by recovering blowdown gas obtained from the reactor in which the methanol formation takes place and subjecting the recovered gas to pressure swing absorption so as to extract hydrogen therefrom, and wherein gas remaining after hydrogen extraction is recycled as a feed to the Catalytic Partial Oxidation.

In another aspect, the invention pertains to a process for the production of synthesis gas from a CO₂-containing hydrocarbon feed, the process comprising subjecting said hydrocarbon feed to Catalytic Partial Oxidation (CPO) in the presence of a molar excess of H₂O, the process being conducted off-shore in a floating production unit.

### Brief description of the drawings

Fig. 1 schematically shows a method according to the prior art for the production of synthesis gas for methanol manufacturing.
Fig. 2 schematically shows an embodiment applicable to be used in the present invention, wherein synthesis gas for methanol production is obtained.
Fig. 3 schematically shows an embodiment applicable to be used in the invention in which pre reforming is adopted.

### Detailed description of the invention

The present invention is based on the judicious insight that catalytic partial oxidation (CPO) has particular benefits for the production of synthesis gas, and particularly for the production of methanol therefrom. Accordingly, CPO is employed for treating hydrocarbon streams that contain a relatively high amount of CO₂, whereby a relatively high amount of steam (H₂O) is employed. Thereby, in deviation from the typical beliefs in the art, it is possible to avoid a need to produce synthesis gas having an R ratio around 2.

As mentioned above, an issue with the conventional production of synthesis gas, in respect of methanol production, is that the carbon to hydrogen ratio is too low. In the method of the present invention, however, a synthesis gas mixture is obtained that has a relatively high proportion of carbon. As a result, the composition of the gas mixture can be easily adjusted for the stoichiometry of methanol production, by the addition of hydrogen prior to methanol formation.

It is particularly found that the process of the invention is suitable for the floating production of methanol, such as by an FPSO process.

Catalytic partial oxidation (CPO) involves a reaction of hydrocarbons with steam and oxygen in the presence of a catalyst. The term CPO as used in the present invention is intended to specifically refer (as often done in the art) to SCT-CPO. CPO is known to the skilled person. SCT-CPO refers to Short Contact Time Catalytic Partial Oxidation. The terms CPO and SCT-CPO are used herein interchangeably. This CPO reaction takes place in a reactor under the influence of a catalyst at residence times between 10⁻² to 10⁻⁴ and with typical catalyst surface contact times around 10⁻⁶ s⁻¹. These contact time correspond to typical space velocities of 100,000 to 250,000 hr⁻¹, preferably 100,000 to 200,000 hr⁻¹. Catalysts employed for SCT-CPO comprise Ni, Pd, Pt, Rh, or Ru. The reaction takes place at catalyst surface temperatures above 950°C, preferably above 1000°C. By employing said short contact times and high catalyst surface temperatures the formation of CO is highly favored and the formation of carbon or CO2 is suppressed. This leads to a highly favorable synthesis gas composition. A reference to CPO is (a) L. Basini, Catalysis Today 117 (2006) 384-393. Other references include (b) L. Basini, K. Aasberg-Petersen, A. Guarinoni, M. Oestberg, Catalysis Today (2001) 64, 9-20 "Catalytic Partial Oxidation of Natural Gas at Elevated Pressure and Low Residence Time"; (c) H. Hickman, L.D. Schmidt, J. Catal. 138 (1992) 267; (d) D. Hichman, L.D. Schmidt Science, 259 (1993) 343; (e) L. Basini, G. Donati WO 97/37929; (f) Sanfilippo, Domenico; Basini, Luca; Marchionna, Mario; EP-640559; (g) D. Schaddenhorst, R.J. Schoonebeek; WO 00/00426; (h) K.L. Hohn, L.D. Schmidt, S. Reyes, J.S. Freeley, WO 01/32556; (i) A.M. Gaffney, R. Songer, R. Ostwald, D. Corbin, WO 01/36323.

According to the present invention, a hydrocarbon containing feed is provided. Any hydrocarbon containing feed normally suitable for steam reforming can be used. Preferably, the feed contains light hydrocarbons such as C₁₋₄ alkanes, *e.g.* methane, ethane, *etc.* Whilst this does not exclude the presence of higher alkanes, it is preferred that the major component of the feed consists of C₁₋₄ alkanes. More preferably, the feed mainly consists of C₁₋₄ alkanes, such as more than 95 wt.% C1-4 alkanes, e.g. more than 98 wt.%.

More preferably, the feed contains, as a major component, methane or a gas containing substantial amounts of methane, preferably natural gas. The invention is particularly suited for hydrocarbon feeds containing carbon dioxide. In accordance with the invention, the hydrocarbon feed comprises carbon dioxide in a range of, generally, 1.0 wt.% to 20 wt.%, such as 2 wt.% to 10 wt.%, such as 2 wt.% to 5 wt.%. In an interesting embodiment, the feed comprises relatively high amounts of carbon dioxide, such as more than 5 wt.%, particularly more than 15 wt. %.

Most preferably, the hydrocarbon feed consists substantially of one or more C₁₋₄ alkanes and carbon dioxide, with possible other components being minor components up to 5 wt.%, preferably below 2 wt.%, more preferably below 1 wt.%.

It is preferred to use a desulfurized feed. Therefore, if needed, the hydrocarbon containing feed can be subjected to a desulfurization step. Methods of accomplishing desulfurization are readily known to the skilled person.

In the art of methanol production it is customary to use hydrocarbon feeds from which CO₂ has been removed, or at least reduced to a lower content, such as up to 2 wt.%. This normally serves to obtain a proper R ratio as discussed above. In the present invention, however, a good use is made of the presence of carbon dioxide, by allowing the CPO reaction for a CO₂-containing hydrocarbon feed to take place in the presence of a relatively high amount of steam.

As a result, the process of the present invention is particularly suitable for being based on natural gas rich in CO₂. In this case, the reaction can be represented as follows (Eq. 1) :

0.8 CH₄+ 0.45 O₂+0.2 CO₂+1.42 H₂O → 1.65 H₂ + 0.58 CO + 0.40 CO₂ +1.33 H₂O +0.02CH₄.

An advantage of avoiding the need for the synthesis gas to have an R ratio around 2, is that no measures need to be taken to remove CO₂ from the hydrocarbon feed. This is of particular benefit in the event of off-shore production, such as the aforementioned FPSO process, as no off-shore removal of carbon dioxide needs to be conducted. A further advantage is that the methanol forming process can be conducted in such a way as to minimize the emission of carbon dioxide, since blowdown gas from methanol synthesis can be recycled back to the CPO.

In this respect, the process of the invention involves the following steps:
- the synthesis gas mixture is subjected to methanol-forming conditions together with an additional feed of hydrogen (H2);
- the additional hydrogen feed is obtained by recovering blowdown gas obtained from the reactor in which the methanol formation takes place and subjecting the recovered gas to pressure swing absorption so as to extract hydrogen therefrom; ,
- gas remaining after hydrogen extraction is recycled as a feed to the Catalytic Partial Oxidation.

Moreover, the present process avoids the need for steam reforming. As discussed above, this presents a particular advantage in the event of FPSO.

The CPO reaction is typically performed in the presence of a metal catalyst. The catalytic metal is preferably a Group VIII noble metal, e.g., platinum, iridium, rhodium, osmium, ruthenium, although nickel may also be used as the catalytic metal. The oxygen used in the catalytic partial oxidation process may be pure or substantially pure oxygen or an oxygen containing gas, *e.g.,* air, or a mixture of oxygen with an inert gas. Substantially pure oxygen (that is, containing more than 99% oxygen) is particularly preferred, and pure oxygen containing more than 99.9% oxygen is still more preferred.

As a result of the CPO reaction, a gas mixture comprising hydrogen (H₂) carbon monoxide (CO) and carbon dioxide (CO₂) is formed. The gas mixture is rapidly cooled so as to be quenched, preferably direct injection of water into the gas stream (i.e., by a direct water quencher). More preferably, this direct water quencher is routed to a methanol reactor. There the quenched gas mixture is combined, preferably before it enters the reactor, with an H₂ stream in order to adjust the R value to the desired one. As mentioned above, the process of producing synthesis gas applied in the invention advantageously results in a gas mixture having an R value preferably in a range of from 1.7 to 1.9. This R value can be easily adjusted, if desired, to a more ideal stoichiometric ratio for the formation of methanol, which is preferably higher than 1.9, and up to 2.1. The H₂ is obtained by recovering it from the blowdown of the methanol reactor.

Accordingly, in one embodiment, part of the synthesis gas mixture is subjected to hydrogen purification, preferably by pressure swing adsorption (PSA), to separate CO₂ and obtain hydrogen with a purity higher than 99%. The purge gas obtained during hydrogen purification, e.g. in a PSA unit, can suitably be supplied to the CPO reactor or added to the hydrocarbon containing feed. The PSA unit thus has an inlet for synthesis gas, and an outlet for hydrogen which is in fluid communication with the main flow of synthesis gas into the methanol reactor.

The blowdown from the methanol reactor is led to a PSA unit, which has an outlet for hydrogen gas, said outlet being in fluid communication with the main flow of synthesis gas into the methanol reactor.

In a pre-reformer, higher hydrocarbons (higher than C1) are converted into methane, which makes the feed more uniform. Another advantage is that oxygen consumption in the CPO reactor is thereby minimized. Adiabatic pre-reforming can be used for pre-reforming. In the pre-reforming the S/C ratio is preferably from 1.5 to 2, more preferably from 1.6 to 1.7.

To ensure a particularly effective conversion in the pre-reforming, the feed can be preheated to the pre-reforming temperature which is, preferably, in the range of 250-600°C, more preferably 450-550°C and in particular about 500°C. To preheat the pre-reforming feed, the heat of process streams elsewhere in the system can be used. For example, the preheating can conveniently be done by exchanging heat with a stream leaving the direct water quencher.

The pre-reforming preheater may be installed downstream of the direct quencher or in a separate heater. In the event that the preheater is downstream of the quencher, it uses the quenched mixture from the CPO and is also heating up the feed (upstream) to the pre-reformer. This preheater is a heat exchanger which exchanges heat between the two streams: feed to pre-reformer and quenched mixture from the CPO.

The present invention applies a method for producing synthesis gas from a hydrocarbon containing CO₂ feed. The method comprises the steps (a) and (b) previously described to obtain the synthesis gas used to produce methanol.

In an interesting embodiment, particularly in the event that the hydrocarbon feed has more than 5 wt.% of CO₂, the hydrocarbon containing feed, or at least part of it, is preferably subjected to pre-reforming. Where "part of a feed" is mentioned, it is understood to encompass more than 0% but less than 100 % of the feed, hence not including the end points "0%" and "100%". For example, the part can be 0.1-99.9%, or 1-99% of the feed, or any other value not being 0% and 100%. In a pre-reformer, higher hydrocarbons (higher than methane) are converted into methane, which makes the feed more uniform. This pre-reforming is particularly beneficial in the event that the hydrocarbon feed comprises a relatively high amount (such as more than 5 wt.%) of CO₂. This can be explained with reference to the fact that in the presence of more CO₂, also more H₂ is desired in order to avoid reaction to CO. This H₂ is generated in the pre-reforming step.

Another advantage is that oxygen consumption in the CPO reactor is thereby minimized. Adiabatic pre-reforming can be used for pre-reforming. In the pre-reforming the steam-to-carbon molar ratio is preferably from 1.5 to 2, more preferably from 1.6 to 1.7.

To ensure a particularly effective conversion in the pre-reforming, the feed can be preheated to the pre-reforming temperature which is, preferably, in the range of 250-600°C, more preferably 450-550°C and in particular about 475°C. To preheat the pre-reforming feed, the heat of process streams elsewhere in the system can be used. For example, the preheating can conveniently be done by exchanging heat with a stream leaving a process gas boiler. In a process gas boiler, a high-temperature stream leaving the CPO reactor is cooled down and can be used to preheat the pre-reforming feed in a gas-gas exchanger. This cooling down is not done by water quenching, but by gas-gas heat exchange. This is still done rapidly, so as to quench the gas.

The feed supplied to the CPO reactor is preferably preheated to a temperature of 200-500°C, preferably 350-450°C and in particularly to a temperature of about 400°C. At these temperatures, the supply of oxygen to the CPO reactor is minimized. This also reduces the costs for the air separation unit (ASU), in case the latter is used to supply oxygen for the CPO reaction. The hydrocarbon containing CO₂ feed and the oxygen can be in various ratios in the feed gas mixture, depending of the feed composition and of the ratio steam to carbon (S/C) which is also fixed in relation of the composition.

Operable ratios can be easily determined by a person skilled in the art. Usually the O₂/C (oxygen to carbon) ratio is in the range of 0.5-1.5 preferably about 0.65.

The S/C ratio is in the range of 1 to 2 preferably about 1.5.

Other than in the process of WO 2013/062413, the product stream of the reaction according to the present invention does not require, neither in whole nor in part, to be subjected to a water gas shift reaction. This presents a clear advantage of the present invention. Hereby it is noted that the invention, if desired, does not exclude subjecting the gas mixture to a water gas shift reaction, or to another desirable further reaction.

Rather, in accordance with the invention, the gas mixture obtained from the CPO reaction is contacted with water so as to provide a quenched synthesis gas mixture. The quenching serves to cool the gas, and thereby set the equilibrium as obtained under the reaction conditions.

Preferably, the quenching is conducted by direct water injection in to the gas mixture. In the absence of a pre-reformer, the gas is cooled to below 450°C, preferably below 400°C If there is a pre-reformer, the feed of which needs preheating, then the gas is cooled to below 550°C, preferably below 500°C and the gas can be used for heat exchange to preheat the feed.

The amount of water used for quenching will depend on temperature to be achieved, as well as the flow. These are regular items for the skilled perso to adjust in practice without any difficulty. It will be understood that the water added does not change the composition of the gas in terms of R ratio.

The quenched synthesis gas employed herein can be subjected to any further treatment, including but not limited to storage, transportation, and/or further reaction. Particularly, in accordance with an aspect of the invention, the quenched synthesis gas is used as a feedstock in the production of methanol.

To this end, the quenched synthesis gas mixture is subjected to methanol-forming conditions. Any suitable method to produce methanol from synthesis gas can be used. Typically, carbon oxides and hydrogen from the synthesis gas react on a catalyst to produce methanol. The catalyst for this reaction usually contains copper and zinc. The step of actually producing methanol from synthesis gas is well-known to the skilled person.

In another aspect, the invention pertains to a process for the production of synthesis gas from a CO₂-containing hydrocarbon feed, the process comprising subjecting said hydrocarbon feed to Catalytic Partial Oxidation (CPO) in the presence of a molar excess of H₂O, the process being conducted off-shore in a floating production unit.

The present invention will further be described with respect to particular embodiments and with reference to certain drawings but the invention is not limited thereto but only by the claims. Any reference signs in the claims shall not be construed as limiting the scope. If not specifically indicated, all percentages for gases are given by volume. The drawings described are only schematic and nonlimiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun *e.g.* "a" or "an", "the", this includes a plural of that noun unless something else is specifically stated.

In sum, the invention provides a process for the production of methanol from a hydrocarbon feed. Particularly, the process is suitable for a hydrocarbon feed that has not been subjected to the removal of carbon dioxide. The process of the invention comprises the steps of (a) providing a hydrocarbon feed; (b) subjecting the hydrocarbon feed to Catalytic Partial Oxidation (CPO) so as to obtain a synthesis gas mixture; and (c) subjecting the synthesis gas mixture to methanol-forming conditions, so as to obtain methanol. The Catalytic Partial Oxidation is conducted in the presence of a molar excess of H₂O. The process of the invention is particularly suitable for the production of methanol in off-shore circumstances, such as by Floating Production Storage and Offloading (FPSO).

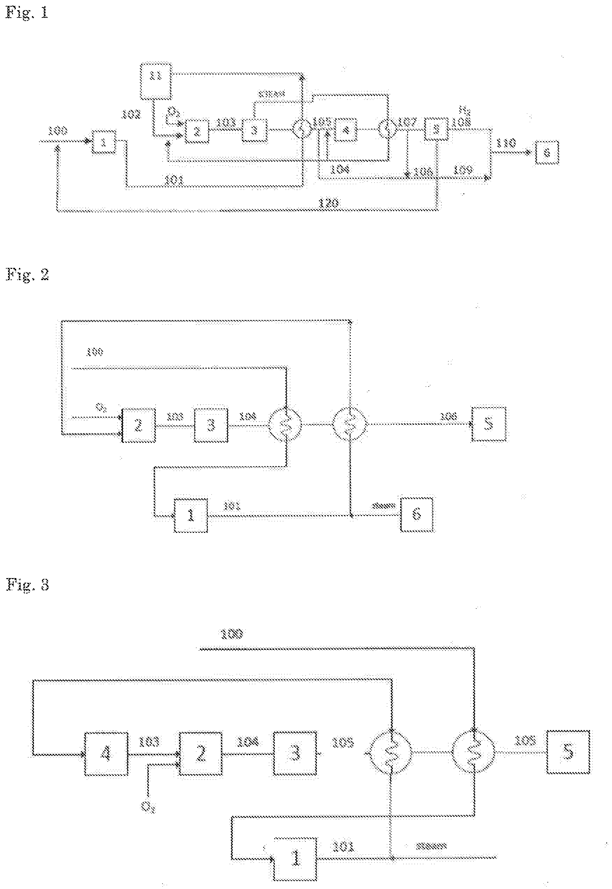

Fig. 1 illustrates a known process for making synthesis gas, in accordance with WO 2013/062413. In this figure, a natural gas feed 100 is desulfurized in a HDS reactor 1. The feed stream 101 is then preheated using heat exchange with a gas stream of a process gas boiler 3 and introduced, after adding steam, to a pre-reforming section 11. To a partially reformed stream 102 pure oxygen is added to carry out conversion of the hydrocarbon feed in a CPO reactor 2, resulting in stream 103.

The stream 103 is cooled in the process gas boiler 3, thereby generating steam and split into two streams 104 and 105. First stream 104 by-passes a WGS reactor 4, while the second stream 105 after steam addition is introduced into the WGS reactor 4. The shifted stream is further split into two streams, 106 and 107. Stream 106 is combined with the unshifted stream 104 to form stream 109, while stream 107 is introduced into a PSA unit 5 or equivalent system to get pure or almost pure H₂ stream 108 and a purge gas stream 120 which may be sent back to the desulfurization unit 1 or in an alternative embodiment directly to the CPO reactor.

Stream 108 is then combined with stream 109 to form stream 110, which is a synthesis gas having an H₂/CO ratio equal to 3 and an R ratio (H₂-CO₂) (CO + CO₂) equal to 2, thereby having a suitable composition to enter a methanol production unit 6.

The splitting ratio for streams 104/105 depends on the feed composition, presence of a pre-reforming section and on the specific synthesis gas application. For methanol application using natural gas as feed and a pre-reforming at 475°C such ratio is preferably 80/20 (by volume). The subsequent ratio 106/107 is preferably 50/50 (vol.).

Fig. 2 shows an embodiment according to the present invention, wherein synthesis gas for methanol production is obtained. In this figure a natural gas feed 100 is preheated and desulfurized in a HDS reactor 1 after being preheated downstream of the CPO reactor 2. To the desulfurized feed 101 steam is added and the resulting stream is preheated before pure oxygen is added to carry out the conversion of the hydrocarbon feed in a CPO reactor 2 resulting in stream 103.

The stream 103 is directly cooled through a direct water quench 3 producing stream 104.

Depending on the steam/carbon ratio selected which in turn depends on the CO₂ content of the natural gas a process gas boiler may be installed to raise the process steam.

Stream 104 once water is condensed has a composition suitable to enter a methanol production unit 5.

Fig. 3 shows an embodiment of the invention in which pre reforming is adopted when the amount of CO₂ is relatively high, such as more than 5 wt.% such as more than 10 wt.%. Such pre-reforming is conducted prior to the CPO reaction. In this figure a natural gas feed 100 is preheated downstream the CPO reactor 2 and desulfurized in a HDS reactor 1. To the desulfurized feed 101 steam is added and the resulting stream 102 is fed to the pre-reforming reactor 4 where the hydrocarbon feed is partially converted in a synthesis gas, 103. After stream 103 is routed to the CPO reactor 2 to totally convert the feed into synthesis gas 104.

The stream 104 is cooled by direct water injecting 3 resulting in stream 105.

Stream 105, once water is condensed, has a composition suitable to enter a methanol production unit 5.

## Claims

1. A process for the production of methanol from a hydrocarbon feed, the process comprising the steps of:
(a) providing a hydrocarbon feed;
(b) subjecting the hydrocarbon feed to Catalytic Partial Oxidation (CPO) so as to obtain a synthesis gas mixture;
(c) subjecting the synthesis gas mixture to methanol-forming conditions together with an additional feed of hydrogen (H2), so as to obtain methanol;
wherein the hydrocarbon feed is a CO₂-containing hydrocarbon feed and wherein the Catalytic Partial Oxidation is conducted in the presence of a molar excess of H₂O, and wherein the additional hydrogen feed is obtained by recovering blowdown gas obtained from the reactor in which the methanol formation takes place and subjecting the recovered gas to pressure swing absorption so as to extract hydrogen therefrom, and wherein gas remaining after hydrogen extraction is recycled as a feed to the Catalytic Partial Oxidation.

2. A process according to claim 1, wherein step (a) comprises:
(a1) providing a hydrocarbon feed comprising alkanes higher than methane,;
(a2) subjecting the hydrocarbon feed to pre-reforming so as to convert alkanes higher than methane to methane.

3. A process according to claim 1 or 2, wherein step (b) comprises:
(b1) subjecting the hydrocarbon feed to a desulfurization step so as to obtain a desulfurized hydrocarbon feed;
(b2) subjecting the desulfurized hydrocarbon feed to CPO.

4. A process according to any one of the preceding claims, wherein step (c) comprises:
(c1) subjecting the synthesis gas mixture to contact with water so as to obtain a quenched synthesis gas;
(c2) subjecting the quenched synthesis gas to methanol-forming conditions.

5. A process according to claim 4, wherein the quenching step (c1) is conducted by direct water injection into the synthesis gas mixture.

6. A process according to any one of the preceding claims, wherein the hydrocarbon feed comprises carbon dioxide in a range of 2.0 wt.% to 20 wt.%, preferably of from 2 wt.% to 10 wt.%.

7. A process according to claim 6, wherein the hydrocarbon feed comprises carbon dioxide in a range of 2 wt.% to 5 wt.%.

8. A process according to claim 6, wherein the hydrocarbon feed comprises more than 5 wt.% of carbon dioxide.

9. A process according to any one of the preceding claims, wherein the major component of the hydrocarbon feed consists of C₁₋₄ alkanes, and preferably the feed consists of at least 95% of C₁₋₄ alkanes.

10. A process according to any one of the preceding claims, conducted off-shore in a floating production unit.

11. A process for the production of synthesis gas from a CO₂-containing hydrocarbon feed, the process comprising subjecting said hydrocarbon feed to Catalytic Partial Oxidation (CPO) in the presence of a molar excess of H₂O, the process being conducted off-shore in a floating production unit.

12. A floating off-shore production unit for the production of methanol, comprising;
- a Catalytic Partial Oxidation (CPO) reactor having an inlet for hydrocarbon feed and an outlet for a synthesis gas mixture,
- a methanol synthesis reactor having an inlet connected to said outlet for synthesis gas mixture of said CPO reactor, and an outlet for blowdown gas,
- a Pressure Swing Absorption (PSA) unit having an inlet connected to said outlet for blowdown gas of said methanol synthesis reactor, and an outlet for obtained hydrogen connected to an inlet of said methanol synthesis reactor, and an outlet for remaining gas connected to an inlet of said CPO reactor.

## Patentansprüche

1. Verfahren zur Herstellung von Methanol aus einem Kohlenwasserstoffeinsatz, das Verfahren umfassend die Schritte von:
(a) Bereitstellen eines Kohlenwasserstoffeinsatzes;
(b) Unterziehen des Kohlenwasserstoffeinsatzes einer katalytischen Teiloxidation (CPO), um ein Synthesegasgemisch zu erhalten;
(c) Unterziehen des Synthesegasgemischs Methanol-bildenden Bedingungen zusammen mit einem zusätzlichen Einsatz von Wasserstoff (H2), um Methanol zu erhalten;
wobei der Kohlenwasserstoffeinsatz ein CO₂-haltiger Kohlenwasserstoffeinsatz ist und wobei die katalytische Teiloxidation in Gegenwart eines molaren Überschusses an H₂O durchgeführt wird, und wobei der zusätzliche Wasserstoffeinsatz durch Zurückgewinnen von Abblasegas, erhalten aus dem Reaktor, in dem die Methanolbildung stattfindet, und Unterziehen von zurückgewonnenem Gas einer Druckwechselabsorption, um Wasserstoff daraus zu extrahieren, erhalten wird, und wobei das nach der Wasserstoffextraktion verbleibende Gas als Einsatz zur katalytischen Teiloxidation zurückgeführt wird.

2. Verfahren nach Anspruch 1, wobei Schritt (a) umfasst:
(a1) Bereitstellen eines Kohlenwasserstoffeinsatzes, umfassend höhere Alkane als Methan;
(a2) Unterziehen des Kohlenwasserstoffeinsatzes einer Vorreformierung, um Alkane höher als Methan, in Methan umzuwandeln.

3. Verfahren nach Anspruch 1 oder 2, wobei Schritt (b) umfasst:
(b 1) Unterziehen des Kohlenwasserstoffeinsatzes einem Entschwefelungsschritt, um einen entschwefelten Kohlenwasserstoffeinsatz zu erhalten;
(b2) Unterziehen des entschwefelten Kohlenwasserstoffeinsatzes einer CPO.

4. Verfahren nach einem der vorhergehenden Ansprüche, wobei Schritt (c) umfasst:
(c1) Unterziehen des Synthesegasgemisches dem Kontakt mit Wasser, um ein gequenchtes Synthesegas zu erhalten;
(c2) Unterziehen des gequenchten Synthesegases Methanol-bildenden Bedingungen.

5. Verfahren nach Anspruch 4, wobei der Quenchschritt (c1) durch direkte Wassereinspritzung in das Synthesegasgemisch durchgeführt wird.

6. Verfahren nach einem der vorhergehenden Ansprüche, wobei der Kohlenwasserstoffeinsatz Kohlendioxid in einem Bereich von 2,0 Gew.-% bis 20 Gew.-%, vorzugsweise von von 2 Gew.-% bis 10 Gew.-%, umfasst.

7. Verfahren nach Anspruch 6, wobei der Kohlenwasserstoffeinsatz Kohlendioxid in einem Bereich von 2 Gew.-% bis 5 Gew.-% umfasst.

8. Verfahren nach Anspruch 6, wobei der Kohlenwasserstoffeinsatzmehr als 5 Gew.-% Kohlendioxid umfasst.

9. Verfahren nach einem der vorhergehenden Ansprüche, wobei die Hauptkomponente des Kohlenwasserstoffeinsatzes aus C1-4-Alkanen, und der Einsatz vorzugsweise zu wenigstens 95 % aus C₁₋₄-Alkanen besteht.

10. Verfahren nach einem der vorangehenden Ansprüche, das off-shore in einer schwimmenden Produktionseinheit durchgeführt wird.

11. Verfahren zur Herstellung von Synthesegas aus einem CO₂haltigen Kohlenwasserstoffeinsatz, das Verfahren umfassend Unterziehen des Kohlenwasserstoffeinsatzes einer katalytischen Teiloxidation (CPO) in der Gegenwart eines molaren Überschusses an H₂O umfasst, wobei das Verfahren off-shore in einer schwimmenden Produktionseinheit durchgeführt wird.

12. Eine schwimmende Offshore-Produktionseinheit zur Herstellung von Methanol, umfassend;
- einen Reaktor zur katalytischen Teiloxidation (CPO) mit einem Einlass für einen Kohlenwasserstoffeinsatz und einem Auslass für ein Synthesegasgemisch,
- einen Reaktor zur Methanolsynthese mit einem Einlass, verbunden mit dem Auslass für das Synthesegasgemisch des CPO-Reaktors, und einem Auslass für Abblasegas,
- eine Druckwechselabsorptions- (PSA-)einheit mit einem Einlass, verbunden mit dem Auslass für Abblasegas des Reaktors zur Methanolsynthese verbunden ist, und einem Auslass für erhaltenen Wasserstoff, verbunden mit einem Einlass des Reaktors zur Methanolsynthese, und einem Auslass für das verbleibende Gas, verbunden mit einem Einlass des CPO-Reaktors.

## Revendications

1. Procédé pour la production de méthanol à partir d'une charge d'hydrocarbures, le procédé comprenant les étapes de :
(a) obtention d'une charge d'hydrocarbures ;
(b) soumission de la charge d'hydrocarbures à une oxydation partielle catalytique (CPO) de façon que soit obtenu un mélange de gaz de synthèse ;
(c) soumission du mélange de gaz de synthèse à des conditions de formation de méthanol conjointement avec une charge additionnelle d'hydrogène (H2), de façon que soit obtenu du méthanol ;
dans lequel la charge d'hydrocarbures est une charge d'hydrocarbures contenant du CO₂ et dans lequel l'oxydation partielle catalytique est effectuée en présence d'un excès molaire de H₂O, et
dans lequel la charge d'hydrogène additionnelle est obtenue par récupération de gaz de chasse obtenu à partir du réacteur dans lequel la formation de méthanol a lieu, et soumission du gaz récupéré à une absorption à pression modulée de façon que l'hydrogène en soit extrait, et
dans lequel le gaz restant après l'extraction d'hydrogène est recyclé en tant que charge vers l'oxydation partielle catalytique.

2. Procédé selon la revendication 1, dans lequel l'étape (a) comprend :
(a1) l'obtention d'une charge d'hydrocarbures comprenant des alcanes supérieurs au méthane ;
(a2) la soumission de la charge d'hydrocarbures à un pré-reformage de façon que les alcanes supérieurs au méthane soient convertis en méthane.

3. Procédé selon la revendication 1 ou 2, dans lequel l'étape (b) comprend :
(b1) la soumission de la charge d'hydrocarbures à une étape de désulfuration de façon que soit obtenue une charge d'hydrocarbures désulfurée ;
(b2) la soumission de la charge d'hydrocarbures désulfurée à une CPO.

4. Procédé selon l'une quelconque des revendications précédentes, dans lequel l'étape (c) comprend :
(c1) la soumission du mélange de gaz de synthèse à un contact avec de l'eau de façon que soit obtenu un gaz de synthèse noyé ;
(c2) la soumission du gaz de synthèse noyé à des conditions de formation de méthanol.

5. Procédé selon la revendication 4, dans lequel l'étape de noyage (c1) est effectuée par injection directe d'eau dans le mélange de gaz de synthèse.

6. Procédé selon l'une quelconque des revendications précédentes, dans lequel la charge d'hydrocarbures comprend du dioxyde de carbone à raison de 2,0 % en poids à 20 % en poids, de préférence de 2 % en poids à 10 % en poids.

7. Procédé selon la revendication 6, dans lequel la charge d'hydrocarbures comprend du dioxyde de carbone à raison de 2 % en poids à 5 % en poids.

8. Procédé selon la revendication 6, dans lequel la charge d'hydrocarbures comprend plus de 5 % en poids de dioxyde de carbone.

9. Procédé selon l'une quelconque des revendications précédentes, dans lequel le composant majeur de la charge d'hydrocarbures consiste en alcanes en C₁₋₄, et de préférence la charge consiste en au moins 95 % d'alcanes en C₁₋₄.

10. Procédé selon l'une quelconque des revendications précédentes, effectué offshore dans une unité de production flottante.

11. Procédé pour la production de gaz de synthèse à partir d'une charge d'hydrocarbures contenant du CO₂, le procédé comprenant la soumission de ladite charge d'hydrocarbures à une oxydation partielle catalytique (CPO) en présence d'un excès molaire de H₂O, le procédé étant effectué offshore dans une unité de production flottante.

12. Unité de production offshore flottante pour la production de méthanol, comprenant :
- un réacteur d'oxydation partielle catalytique (CPO) ayant une entrée pour une charge d'hydrocarbures et une sortie pour un mélange de gaz de synthèse,
- un réacteur de synthèse de méthanol ayant une entrée connectée à ladite sortie pour un mélange de gaz de synthèse dudit réacteur CPO, et une sortie pour le gaz de chasse,
- une unité d'absorption à pression modulée (PSA) ayant une entrée connectée à ladite sortie pour le gaz de chasse dudit réacteur de synthèse de méthanol, et une sortie pour l'hydrogène obtenu connectée à une entrée dudit réacteur de synthèse de méthanol, et une sortie pour le gaz restant connectée à une entrée dudit réacteur CPO.
